# EUROPÄISCHE PATENTSCHRIFT

(11) **EP 1 637 468 B1**
(45) Veröffentlichungstag und Bekanntmachung des Hinweises auf die Patenterteilung: **16.07.2008**
(21) Anmeldenummer: 05020402.3
(22) Anmeldetag: 19.09.2005
(51) Int. Cl.: B65G 47/14, A61J 7/02, B65B 35/08

(54) **Fördervorrichtung für Objekte von fester oder gelartiger Konsistenz sowie Verfahren zur Förderung derartiger Objekte**
Feeding device for solid or gel-type objects and method of feeding such objects
Dispositif de transfert d'objets de consistence solide ou gélifiée et méthode de transfert de tels objets

(30) Priorität: 20.09.2004 DE 102004045847
(43) Veröffentlichungstag der Anmeldung: 22.03.2006
(73) Patentinhaber: Kraemer, Thilo, 64291 Darmstadt (DE)
(72) Erfinder: Kraemer, Thilo, 64291 Darmstadt (DE)
(74) Vertreter: Mierswa, Klaus

(56) Entgegenhaltungen:
- CH-A5- 596 064
- DE-A1- 10 323 670
- FR-A- 2 689 092
- US-A- 1 495 178
- US-A- 2 507 883
- US-A- 2 768 743
- US-A- 5 810 198
- US-A- 5 826 697
- US-B1- 6 471 093

## Beschreibung

### Technisches Gebiet:

Die Erfindung betrifft eine Fördervorrichtung für Objekte von fester oder gelartiger Konsistenz gemäß dem Oberbegriff des Anspruchs 1 sowie ein Verfahren zur Förderung derartiger Objekte gemäß dem Oberbegriff des Anspruchs 17.

### Stand der Technik:

Im Stand der Technik sind zahlreiche Fördervorrichtungen und Förderverfahren für Objekte bekannt. Gemäß dem Stand der Technik erweist es sich oft als Problem, bei der massenhaften Förderung insbesondere von kleinen Objekten, wie z.B. Tabletten, eine schnelle und sichere Vereinzelung oder Gruppierung der Objekte, insbesondere zum Zweck ihrer Prüfung oder Verpackung, zu erreichen.

Aus der FR-A-2 689 092 ist eine gattungsgemäße Vorrichtung und ein gattungsgemäßes Verfahren zum gruppenweisen Abzählen von kleinen Kugeln und dergleichen und zur Gruppierung derselben in einem Gefäß bekannt. Die Kugeln gelangen aus einem Vorrat in einen schrägstehenden tellerartigen Behälter, welcher in Rotation versetzt werden kann. Der Behälter weist in seinem Boden mehrere Gruppen von Löchern auf, wobei der Durchmesser jedes Lochs etwas größer ist als der Durchmesser der abzuzählenden Kugeln. Unter dem Boden ist eine feststehende Platte mit einer Ausgangsöffnung angeordnet. Eine Gruppe von Kugeln, welche in die Löcher einer Lochgruppe fällt, wird von der Rotation des Behälters für einen Teil einer Umdrehung mitgenommen, fällt bei Erreichen der Ausgangsöffnung durch dieselbe hindurch aus dem Behälter heraus und gelangt in ein Aufnahmegeäß. Auf diese Weise wird eine Gruppe von Kugeln, bestehend aus einer bestimmten Zahl von Kugeln, aus dem Vorrat ausgesondert und in das Aufnahmegefäß befördert.

### Technische Aufgabe:

Der Erfindung liegt die Aufgabe zu Grunde, ein Fördereinrichtung für Objekte von fester oder gelartiger Konsistenz, insbesondere für pharmazeutische Objekte, wie Tabletten, Pillen oder Oblongs, sowie ein Verfahren zur Förderung derartiger Objekte, zu schaffen, welche eine schnelle und sichere Vereinzelung oder Gruppierung der Objekte ermöglichen, beispielsweise um eine definierte Anzahl solcher Objekte aus einem Vorrat auszusondern, etwa zu Verpackungszwecken, oder z.B. um die Objekte einzeln einer automatischen Prüfung oder Vermessung zu unterziehen.

Die Aufgabe wird erfindungsgemäß gelöst durch eine Fördervorrichtung für Objekte von fester oder gelartiger Konsistenz, insbesondere für pharmazeutische Objekte, wie Tabletten, Pillen oder Oblongs, mit einem drehbar gelagerten Förderrad, einem Drehantrieb und einer unteren Abdeckung, wobei
- das Förderrad durch einen Radboden sowie eine den Innenraum des Förderrades peripher umschließende Seitenwandung becher-, schüssel- oder muldenförmig ausgebildet ist, wobei der Innenraum zur Aufnahme der Objekte vor deren Vereinzelung dient,
- der Drehantrieb imstande ist, das Förderrad um dessen Radachse zu drehen,
- die Richtung der Radachse des Förderrades einen vorgegebenen Kippwinkel ψ von 0° bis 90° zur Senkrechten einnimmt, wodurch sich die Objekte im Bereich des tiefsten Punktes des Innenraumes sammeln,
- die untere Abdeckung auf der vom Innenraum abgewandten Seite des Radbodens parallel zu diesem angeordnet ist und nicht an der Rotation des Förderrades teilzunehmen imstande ist,
- die untere Abdeckung entweder am Radboden anliegt oder in einer solchen Distanz von diesem angeordnet ist, welche kleiner ist als der minimale Durchmesser der Objekte,
- der Radboden in einem vorgegebenen Abstand von der Radachse wenigstens ein Mitnahmeloch aufweist, dessen Lage und Abmessungen so gewählt sind, dass genau eines der im Bereich des tiefsten Punktes des Innenraumes versammelten Objekte so in das Mitnahmeloch hineinfallen kann, dass es in stabiler Lage am Rand des Mitnahmelochs sowie auf der unteren Abdeckung anliegt und dadurch imstande ist, unter Abrollen oder Gleiten auf der unteren Abdeckung an der Rotation des Förderrades teilzunehmen und auf diese Weise den Bereich des tiefsten Punktes des Innenraumes zu verlassen, und
- die untere Abdeckung einen ersten Teil der zur Radachse parallelen Projektion des Kreises, welchen das Mitnahmeloch um die Radachse beschreibt, abdeckt, und einen zweiten Teil dieser Projektion nicht abdeckt, wodurch das in das Mitnahmeloch hineingefallene Objekt dann nach unten aus dem Mitnahmeloch herausfällt und somit aus der Fördervorrichtung ausgeworfen wird, wenn die zur Radachse parallele Projektion des Mitnahmelochs den zweiten Teil erreicht,
so dass die Fördereinrichtung zur automatischen Vereinzelung der Objekte imstande ist, wobei die Rotationsrichtung des Förderrades umkehrbar ist, und die untere Abdeckung auch einen vom zweiten Teil beabstandeten dritten Teil der zur Radachse parallelen Projektion des Kreises, welchen das Mitnahmeloch um die Radachse beschreibt, nicht abdeckt, so dass das in das Mitnahmeloch oder eines der Zusatz-Mitnahmelöcher hineingefallene Objekt bei Rotation des Förderrades in einer Richtung bei Erreichen des zweiten Teils und bei Rotation des Förderrades in der anderen Richtung bei Erreichen des dritten Teils ausgeworfen wird und somit der Ort des Auswurfes durch Vorgeben der Rotationsrichtung des Förderrades wählbar ist.

Bevorzugt ist der Innenraum an seiner vom Radboden abgewandten Seite offen; alternativ hierzu kann er an der vom Radboden abgewandten Seite abgedeckt sein.

Bevorzugt erstreckt sich der erste Teil über einen Kreisbogen, welcher den Bereich des tiefsten Punktes der zur Radachse parallelen Projektion des Kreises umfaßt.

Gemäß einer Variante erstreckt sich der erste Teil wenigstens über einen Kreisbogen, welcher sich vom tiefsten Punkt des Kreises aus über einen Winkel von wenigstens ±10°, vorzugsweise über einen Winkel von wenigstens ±30° erstreckt.

Bevorzugt ist die Radachse um einen Kippwinkel ψ zwischen 10° und 80°, vorzugsweise zwischen 30° und 60°, gegen die Senkrechte geneigt, so dass sich das Förderrad in Schrägstellung befindet. Insbesondere kann der Kippwinkel ψ verstellbar sein.

Der Radboden weist bevorzugt zusätzlich zu dem Mitnahmeloch ein, mehrere oder viele Zusatz-Mitnahmelöcher auf, deren Abmessungen mit denjenigen des Mitnahmelochs übereinstimmen und welche gemeinsam mit dem Mitnahmeloch alle auf einem Kreis liegen. Gemäß einer abweichenden Variante weist wenigstens ein Teil der Zusatz-Mitnahmelöcher andere Abmessungen auf als das Mitnahmeloch.

Bevorzugt weist die erfindungsgemäße Fördervorrichtung eine Auffangeinrichtung, insbesondere Schlauch, Rohr oder Rinne auf, welche die nach unten aus dem Mitnahmeloch oder einem der Zusatz-Mitnahmelöcher herausgefallenen Objekte zum Zweck der Weiterleitung aufnimmt.

Gemäß einer vorteilhaften Ausführungsform ist der Radboden an seiner dem Innenraum zugewandten Seite konisch, kegelmantelfömig oder konvex ausgebildet.

Bevorzugt weist die erfindungsgemäße Fördervorrichtung einen Vibrator auf, welcher das Förderrad wenigstens zeitweise in Vibration zu versetzen imstande ist.

Die Abmessungen des Mitnahmelochs bzw. des Mitnahmelochs und der Zusatz-Mitnahmelöcher sind bevorzugt so gewählt, dass anstelle jeweils genau eines der Objekte jeweils eine vorgegebene Anzahl von genau n Objekten in das Mitnahmeloch bzw. in das Mitnahmeloch und die Zusatz-Mitnahmelöcher hineinfallen und danach nach unten aus dem Mitnahmeloch bzw. dem Zusatz-Mitnahmeloch herausfallen können, so dass die Fördereinrichtung, anstatt zur automatischen Vereinzelung der Objekte imstande zu sein, zur automatischen Gruppierung der Objekte zu Gruppen von jeweils genau n Objekten imstande ist.

Die Rotationsgeschwindigkeit des Förderrades ist gemäß einer bevorzugten Ausführungsform veränderbar. Bevorzugt ist die Rotation des Förderrades intervallweise anhaltbar und wieder in Gang setzbar.

Gemäß einer vorteilhaften Ausführungsform weist die erfindungsgemäße Fördervorrichtung einen Aufprallsensor auf, mittels welchem die Zahl der ausgeworfenen Objekte zählbar ist.

Gemäß einer vorteilhaften Ausführungsform weist die erfindungsgemäße Fördervorrichtung eine obere Abdeckung auf, welche im Innenraum parallel zum Radboden angeordnet und nicht an der Rotation des Förderrades teilzunehmen imstande ist, entweder am Radboden anliegt oder in einer solchen Distanz von diesem angeordnet ist, welche kleiner ist als der minimale Durchmesser der Objekte, und als Abstreifer für solche im Innenraum befindlichen Objekte dient, welche nicht in das Mitnahmeloch oder eines der Zusatz-Mitnahmelöcher hineingefallen sind.

Der Drehantrieb ist bevorzugt imstande, das Förderrad durch mehrmaliges aufeinanderfolgendes Umschalten der Rotationsrichtung in eine Schaukelbewegung zu versetzen.

Gemäß einer vorteilhaften Ausführungsform ist der Radboden auswechselbar gegen einen solchen mit
- einem Mitnahmeloch von anderen Abmessungen oder,
- Mitnahmelöchern bzw. Zusatz-Mitnahmelöchern von anderen Abmessungen, oder
- Mitnahmelöchern bzw. Zusatz-Mitnahmelöchern von anderer Zahl.

Die erfindungsgemäße Fördervorrichtung kann insbesondere Bestandteil einer Vorrichtung zur Bestimmung wenigstens eines physikalischen oder chemischen Parameters von Objekten, insbesondere von pharmazeutischen Objekten, wie Tabletten, Pillen oder Oblongs, sein.

Die Aufgabe wird ferner erfindungsgemäß gelöst durch ein Verfahren zur Förderung von Objekten von fester oder gelartiger Konsistenz, insbesondere von pharmazeutischen Objekten, wie Tabletten, Pillen oder Oblongs, unter Verwendung eines drehbar gelagerten Förderrades, eines Drehantriebes und einer unteren Abdeckung, wobei
- als Förderrad ein solches verwendet wird, welches durch einen Radboden sowie eine den Innenraum des Förderrades peripher umschließende Seitenwandung becher-, schüssel- oder muldenförmig ausgebildet ist,
wobei der Innenraum zur Aufnahme der Objekte vor deren Vereinzelung dient,
- das Förderrad durch den Drehantrieb um die Radachse des Förderrades gedreht wird, wobei die Richtung der Radachse des Förderrades um einen vogegebenen Kippwinkel ψ von 0° bis 90° zur Senkrechten einnimmt, wodurch sich die Objekte im Bereich des tiefsten Punktes des Innenraumes sammeln,
- als untere Abdeckung eine solche verwendet wird, welche auf der vom Innenraum abgewandten Seite des Radbodens parallel zu diesem angeordnet ist und nicht an der Rotation des Förderrades teilzunehmen imstande ist, und welche entweder am Radboden anliegt oder in einer solchen Distanz von diesem angeordnet ist, welche kleiner ist als der minimale Durchmesser der Objekte,
- als Radboden ein solcher verwendet wird, welcher in einem vorgegebenen Abstand von der Radachse wenigstens ein Mitnahmeloch aufweist, dessen Lage und Abmessungen so gewählt sind, dass genau eines der im Bereich des tiefsten Punktes des Innenraumes versammelten Objekte so in das Mitnahmeloch hineinfallen kann, dass es in stabiler Lage am Rand des Mitnahmelochs sowie auf der unteren Abdeckung anliegt und dadurch imstande ist, unter Abrollen oder Gleiten auf der unteren Abdeckung an der Rotation des Förderrades teilzunehmen und auf diese Weise den Bereich des tiefsten Punktes des Innenraumes zu verlassen, und
- als untere Abdeckung eine solche verwendet wird, welche einen ersten Teil der zur Radachse parallelen Projektion des Kreises, welchen das Mitnahmeloch um die Radachse beschreibt, abdeckt, und einen zweiten Teil dieser Projektion nicht abdeckt, wodurch das in das Mitnahmeloch hineingefallene Objekt dann nach unten aus dem Mitnahmeloch herausfällt und somit aus der Fördervorrichtung ausgeworfen wird, wenn die zur Radachse parallele Projektion des Mitnahmelochs den zweiten Teil erreicht,
so dass mittels der Fördereinrichtung die Objekte automatisch vereinzelt werden. wobei die Rotationsrichtung des Förderrades in bestimmten regelmäßigen oder unregelmäßigen Zeitabständen umgekehrt wird, und als untere Abdeckung eine solche verwendet wird, welche auch einen vom zweiten Teil beabstandeten dritten Teil der zur Radachse parallelen Projektion des Kreises, welchen das Mitnahmeloch um die Radachse beschreibt, nicht abdeckt, so dass das in das Mitnahmeloch oder eines der Zusatz-Mitnahmelöcher hineingefallene Objekt bei Rotation des Förderrades in einer Richtung bei Erreichen des zweiten Teils und bei Rotation des Förderrades in der anderen Richtung bei Erreichen des dritten Teils ausgeworfen wird und somit der Ort des Auswurfes durch Vorgeben der Rotationsrichtung des Förderrades wählbar ist.

Bevorzugt erstreckt sich der erste Teil über einen Kreisbogen, welcher den Bereich des tiefsten Punktes der zur Radachse parallelen Projektion des Kreises umfaßt.

Gemäß einer Variante erstreckt sich der erste Teil wenigstens über einen Kreisbogen, welcher sich vom tiefsten Punkt des Kreises aus über einen Winkel von wenigstens ±10°, vorzugsweise über einen Winkel von wenigstens ±30° erstreckt.

Bevorzugt ist die Radachse um einen Kippwinkel ψ zwischen 10° und 80°, vorzugsweise zwischen 30° und 60°, gegen die Senkrechte geneigt, so dass sich das Förderrad in Schrägstellung befindet. Der Kippwinkel ψ kann insbesondere verstellbar sein.

Als Radboden kann insbesondere ein solcher verwendet werden, welcher zusätzlich zu dem Mitnahmeloch ein, mehrere oder viele Zusatz-Mitnahmelöcher aufweist, deren Abmessungen mit denjenigen des Mitnahmeochs übereinstimmen und welche gemeinsam mit dem Mitnahmeloch alle auf einem Kreis liegen.

Bevorzugt wird eine Auffangeinrichtung, welche insbesondere z.B. ein Schlauch, Rohr oder Rinne sein kann, verwendet, welche die nach unten aus dem Mitnahmeloch oder einem der Zusatz-Mitnahmelöcher herausgefallenen Objekte zum Zweck der Weiterleitung aufnimmt.

Bevorzugt ist der Radboden an seiner dem Innenraum zugewandten Seite konisch, kegelmantelfömig oder konvex ausgebildet.

Bevorzugt wird das Förderrad wenigstens zeitweise durch einen Vibrator in Vibration versetzt.

Die Abmessungen des Mitnahmelochs bzw. des Mitnahmelochs und der Zusatz-Mitnahmelöcher können so gewählt sein, dass anstelle jeweils genau eines der Objekte jeweils eine vorgegebene Anzahl von genau n Objekten in das Mitnahmeloch bzw. in das Mitnahmeloch und die Zusatz-Mitnahmelöcher hineinfallen und danach nach unten aus dem Mitnahmeloch bzw. dem Zusatz-Mitnahmeloch herausfallen können, so dass die Fördereinrichtung, anstatt zur automatischen Vereinzelung der Objekte imstande zu sein, zur automatischen Gruppierung der Objekte zu Gruppen von jeweils genau n Objekten imstande ist.

Gemäß einer Verfahrensvariante wird die Rotationsgeschwindigkeit des Förderrades verändert. Gemäß einer Variante des Verfahrens wird die Rotation des Förderrades intervallweise angehalten und wieder in Gang gesetzt.

Die Zahl der ausgeworfenen Objekte wird gemäß einer Variante mittels eines Aufprallsensors gezählt.

Es kann eine obere Abdeckung verwendet werden, welche im Innenraum parallel zum Radboden angeordnet und nicht an der Rotation des Förderrades teilzunehmen imstande ist, entweder am Radboden anliegt oder in einer solchen Distanz von diesem angeordnet ist, welche kleiner ist als der minimale Durchmesser der Objekte, und als Abstreifer für solche im Innenraum befindlichen Objekte dient, welche nicht in das Mitnahmeloch oder eines der Zusatz-Mitnahmelöcher hineingefallen sind.

Als Drehantrieb kann ein solcher verwendet werden, welcher imstande ist, das Förderrad durch mehrmaliges aufeinanderfolgendes Umschalten der Rotationsrichtung in eine Schaukelbewegung zu versetzen.

Als Radboden kann ein solcher verwendet werden, welcher auswechselbar ist gegen einen solchen mit
- einem Mitnahmeloch von anderen Abmessungen oder,
- Mitnahmelöchern bzw. Zusatz-Mitnahmelöchern von anderen Abmessungen, oder
- Mitnahmelöchern bzw. Zusatz-Mitnahmelöchern von anderer Zahl.

Das erfindungsgemäße Verfahren kann insbesondere Teil eines Verfahrens zur Bestimmung wenigstens eines physikalischen oder chemischen Parameters von Objekten, insbesondere von pharmazeutischen Objekten, wie Tabletten, Pillen oder Oblongs, sein.

### Kurzbeschreibung der Zeichnung, in welcher zeigen:

Figuren 1 bis 4 zeigen jeweils eine Draufsicht auf bestimmte Bestandteile einer erfindungsgemäßen Fördereinrichtung
- Figur 1: das becherartig durch einen Radboden und eine Seitenwandung gebildete Förderrad einer erfindungsgemäßen Fördereinrichtung in Draufsicht, wobei das Förderrad durch einen Drehantrieb in Rotation versetzbar ist,
- Figur 2: das Förderrad und den Drehantrieb von Figur 1, wobei hinter dem Radboden eine untere Abdeckung mit zwei peripheren Aussparungen angeordnet ist, wobei der Radboden halbdurchsichtig dargestellt ist und sich im Bereich des tiefsten Punktes des Innenraums des Förderrades zahlreiche zu fördernde runde Objekte befinden,
- Figur 3: die untere Abdeckung von Figur 2 allein,
- Figur 4: das Förderrad, den Drehantrieb, die untere Abdeckung und die Objekte von Figur 2, wobei zusätzlich vor dem dem Radboden eine durchsichtig dargestellte obere Abdeckung angeordnet ist, und
- Figur 5: eine Ausführungsform einer vollständigen erfindungsgemäßen Fördervorrichtung in Seitenansicht, mit einem im Querschnitt dargestellten Schlauch zur Ausgabe der geförderten Objekte, wobei die Seitenwandung durchsichtig dargestellt ist.

Alle nachfolgenden Figuren sind schematische Darstellungen, welche sich auf beispielhafte Ausführungsformen beziehen.

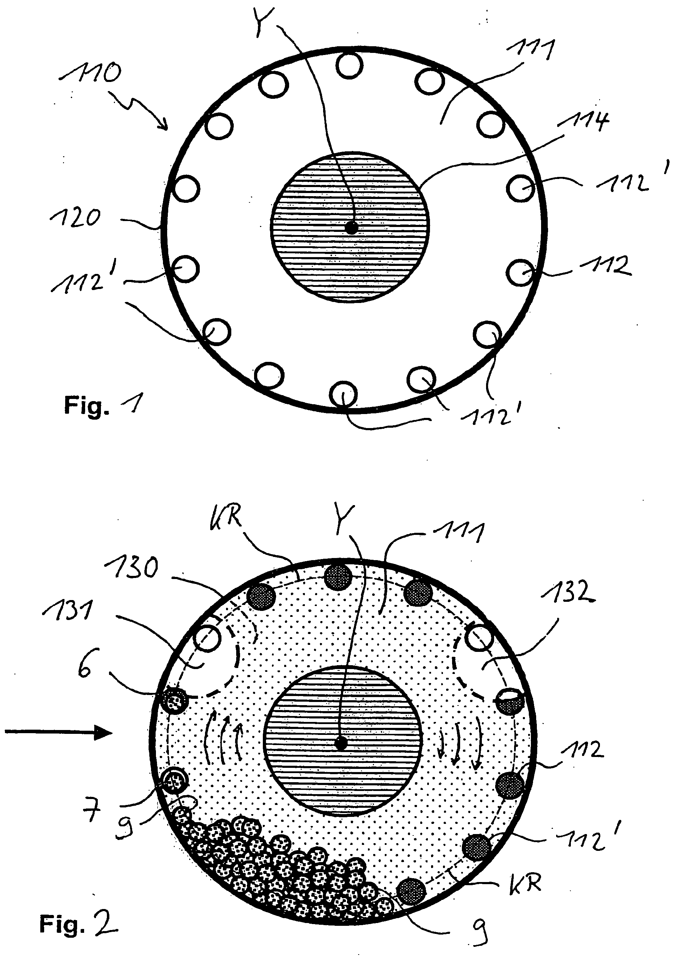

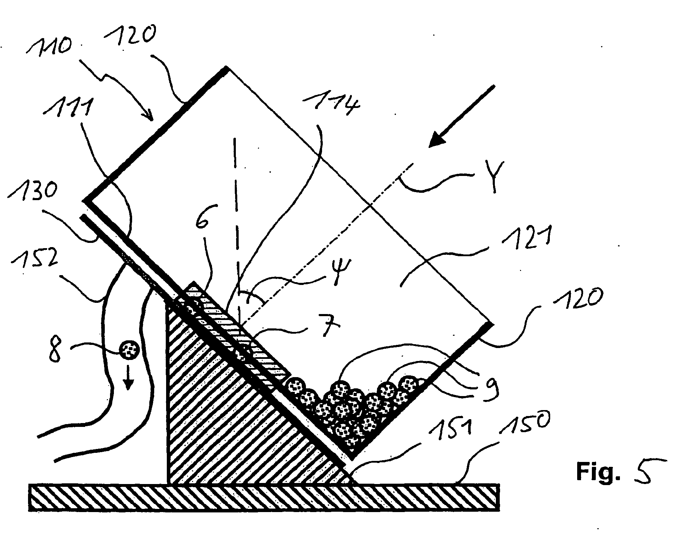

Die Figuren 1 bis 4 zeigen jeweils eine Draufsicht auf bestimmte Bestandteile einer bevorzugten Ausführungsform einer erfindungsgemäßen Fördereinrichtung für Objekte von fester oder gelartiger Konsistenz, d.h. die Objekte können Festkörper oder gelarige Körper sein. Fig. 5 zeigt eine vollständige erfindungsgemäße Fördereinrichtung in Seitenansicht, wobei die Seitenwandung 120 durchsichtig und der Schlauch 152 im Querschnitt dargestellt ist. Die Blickrichtung von Fig. 5 entspricht der Richtung des nach rechts weisenden Pfeils von Fig. 2.

Der von der Seitenwandung 120 umgebene Raum wird im folgenden als "Innenraum" 121 bezeichnet.

Die Fördervorrichtung weist ein drehbar gelagertes Förderrad 110 mit Drehantrieb 114 auf, welcher imstande ist, das Förderrad 110 um dessen Radachse Y zu drehen. Das Förderrad 110 ist durch einen Radboden 111 sowie eine den Innenraum 121 des Förderrades 110 peripher umschließende Seitenwandung 120 becher-, schüssel- oder muldenförmig ausgebildet. Der Innenraum 121 dient zur Aufnahme der zu fördernden Objekte 9 vor deren Vereinzelung. Vorzugsweise ist der Innenraum 121 an seiner vom Radboden 111 abgewandten Seite offen.

Im Beispiel von Fig. 5 ist die Richtung der Radachse Y des Förderrades 110 um einen vorgegebenen Kippwinkel ψ von weniger als 90°, vorzugsweise um 30° bis 60°, z.B. um 45°, gegen die Senkrechte geneigt, so dass sich das Förderrad 110 in Schrägstellung befindet (Fig. 5), wodurch sich die Objekte 9 im Bereich des tiefsten Punktes des Innenraumes 121 sammeln. Einzelne Objekte, welche gerade durch die erfindungemäße Fördereinrichtung aus der Masse der in diesem Bereich versammelte Objekte 9 herausgefördert werden oder bereits herausgefördert wurden, sind mit den Bezugszeichen 6,7 und 8 bezeichnet.

Der Radboden 111 weist in einem vorgegebenen Abstand von der Radachse Y ein Mitnahmeloch 112 sowie zahlreiche Zusatz-Mitnahmelöcher 112' (auch als Zusatzlöcher 112' bezeichnet) auf. Das Förderrad 110 mit dem Radboden 111, der Seitenwandung 120, dem Mitnahmeloch 11 und den Zusatz-Mitnahmelöchern 112' sowie der Drehantrieb 114 sind in Fig. 1 in Draufsicht in Richtung der Drehachse Y dargestellt.

Das Mitnahmeloch 112 und die Zusatz-Mitnahmelöcher 112' werden im folgenden zusammenfassend als "Mitnahmelöcher" oder kurz als "Löcher" bezeichnet.

Vorzugsweise sind alle Löcher 112, 112' mit identischen Abmessungen ausgebildet. Ferner befinden sich vorzugsweise alle Löcher 112, 112' in gleichem Abstand von der Drehachse Y, so dass alle Löcher 112, 112' bzw. deren Mittelpunkte bei Rotation des Förderrades 110 ein- und denselben Kreis KR beschreiben.

Die Fördervorrichtung weist ferner eine untere Abdeckung 130 auf, welche auf der vom Innenraum 121 abgewandten Seite des Bodens 111 parallel oder im wesentlichen zu diesem angeordnet ist und nicht an der Rotation des Förderrades 110 teilzunehmen imstande ist, sondern vielmehr über einen Keil 151 starr mit einer Fußplatte 150 verbunden ist (Fig. 5). Die untere Abdeckung 130 liegt entweder am Radboden 111 an oder in ist, wie im Beispiel von Fig. 5, in einer Distanz vom Radboden 111 angeordnet, welche kleiner ist als der minimale Durchmesser der Objekte 6,7,8,9, so dass keines der Objekte in den Zwischenraum zwischen Radboden 111 und unterer Abdeckung 130 geraten kann.

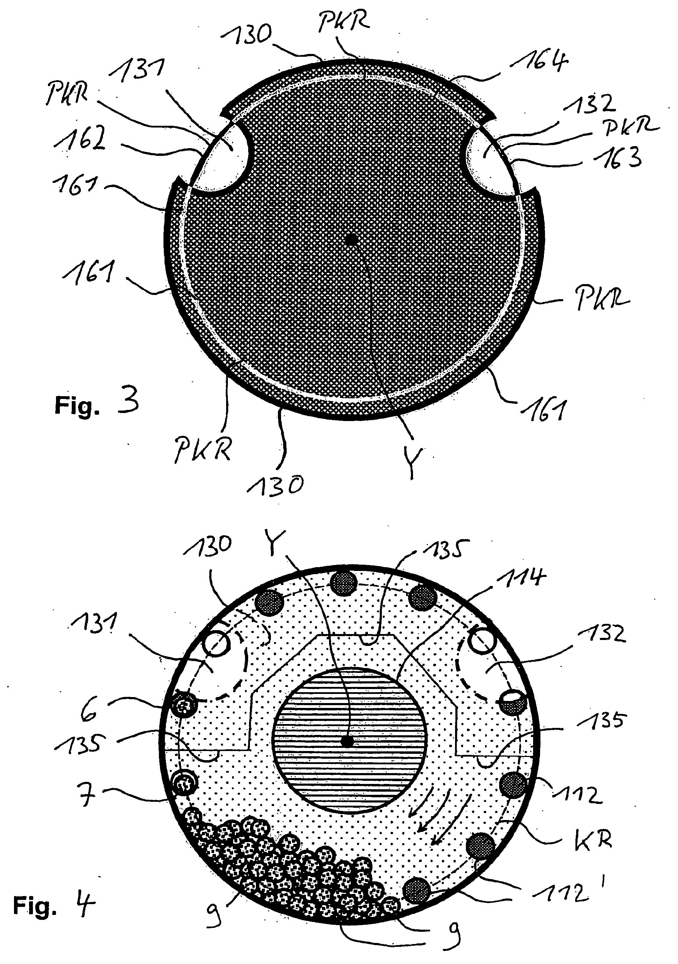

Die untere Abdeckung 130 ist in Fig. 1 nicht dargestellt. In den Figuren 2 und 4 ist der Radboden 111 halbdurchsichtig dargestellt. Die untere Abdeckung 130 befindet sich in diesen Figuren 2 und 4 unter dem Radboden 111 und ist in diesen Figuren durch ein helles Punktmuster dargestellt. Durch die Löcher 112, 112' hindurch ist die untere Abdeckung 130 uneingeschränkt sichtbar und daher dort durch ein dunkleres Punktmuster dargestellt.

Die Lage und Abmessungen der Löcher 112, 112' sind so gewählt, dass je genau eines der im Bereich des tiefsten Punktes des Innenraumes 121 versammelten Objekte 9 dergestalt in das Mitnahmeloch 112 oder eines der Zusatz-Mitnahmelöcher 112' hineinfallen kann, dass das Objekt in stabiler Lage am Rand des betreffenden Mitnahmelochs 112 oder Zusatz-Mitnahmelochs 112' sowie zugleich auch auf der unteren Abdeckung 130 anliegt und dadurch imstande ist, unter Abrollen oder Gleiten auf der unteren Abdeckung 130 bei Rotation des Förderrades 110 an dessen Rotation teilzunehmen und auf diese Weise den Bereich des tiefsten Punktes des Innenraumes 121 zu verlassen. Dies ist in Fig. 2 und Fig. 4 für je zwei Objekte 6,7 exemplarisch dargestellt.

In Fig. 3 ist die untere Abdeckung 130 allein dargestellt. Sie besitzt im hier in Rede stehenden Ausführungsbeispiel im wesentlichen die Form einer Kreisscheibe mit dem Durchmesser des Förderrades 110, wobei der Mittelpunkt der unteren Abdeckung 130 auf der Radachse Y liegt. Die Projektion des Kreises KR in Richtung der Radachse Y auf die Ebene der unteren Abdeckung 130 ist durch einen geschlossenen Kreis PKR veranschaulicht. Ein großer Teil 161 dieser Projektion PKR, im folgenden als erster Teil 161 der Projektion PKR bezeichnet, trifft die untere Abdeckplatte 130. Dasselbe gilt für einen weiteren Teil 164 dieser Projektion PKR, im folgenden als vierter Teil 164 der Projektion PKR bezeichnet. Der erste und der vierte Teil 161, 164 sind in Fig. 3 als weiße Kreisbögen veranschaulicht.

Die untere Abdeckung 130 weist in Abweichung von der Kreisscheibenform zwei Aussparungen 131 und 132 auf, welche sich mit der Projektion PKR überschneiden. Zwei Teile der Projektion PKR, nämlich ein zweiter Teil 162 und ein dritter Teil 163, treffen daher die untere Abdeckung 130 nicht, sondern verfehlen sie auf Grund des Vorhandenseins der Aussparungen 131,132. Diese Teile der Projektion PKR sind in Fig. 3 durch schwarze Kreisbögen 162, 163 veranschaulicht.

Erfindungsgemäß deckt somit die untere Abdeckung 130 einen ersten Teil 161 und einen vierten Teil 164 der Projektion PKR ab und einen zweiten und einen dritten Teil 162,163 dieser Projektion PKR nicht ab, was bedeutet, dass die untere Abdeckung 130 den Radboden 110 gegen die Projektionsrichtung gesehen nur teilweise bedeckt.

Der dritte Teil 163 kann prinzipiell als Teil des zweiten Teils betrachtet oder definiert werden, so dass gemäß dieser Betrachtungsweise oder Definition der zweite Teil seinerseits aus zwei voneinander beabstandeten Teilen 162, 163 besteht. welche gemeinsam den zweiten Teil 162,163 der Projektion PKR bilden, und so dass gemäß dieser Betrachtungsweise oder Definition des Objekt immer dann ausgeworfen wird, wenn die zur Radachse Y parallele Projektion des betreffenden Lochs 112,112' den zweiten Teil 162,163 erreicht.

Die Aussparungen 131,132 sind hierbei so bemessen und angeordnet, das ein in eines der Mitnahmelöcher 112,112' hineingefallenes Objekt 6,7 dann nach unten aus diesem Loch 112,112' herausfällt und somit aus der Fördervorrichtung ausgeworfen wird, wenn die zur Radachse Y parallele Projektion dieses Lochs 112 oder 112' auf die Ebene der unteren Abdeckung 130 eine der Aussparungen 131,132 erreicht. Auf diese Weise ist die Fördereinrichtung erfindungsgemäß zur automatischen Vereinzelung der Objekte imstande. Bei Linksdrehung des Förderrades 110 fällt das Objekt daher durch die Aussparung 131 hindurch heraus, bei Rechtsdrehung des Förderrades 110 fällt das Objekt daher durch die Aussparung 132 hindurch heraus.

Ein auf diese Weise erfindungsgemäß einzeln ausgeworfenenes Objekt 8 ist in Fig. 5 dargestellt. Jedes auf diese Weise ausgeworfene Objekt kann z.B. durch einen Schlauch 152 (in Fig. 5 im Querschnitt dargestellt) einer (nicht gezeigten) Vorrichtung bzw. Prüfmaschine zur Bestimmung wenigstens eines physikalischen oder chemischen Parameters des Objekts, wie Gewicht, Härte, Dicke, Länge, Durchmesser, Löslichkeit, Schadstoffkonzentration, zugeführt werden. Die erfindungsgemäße Fördervorrichtung kann also in einer derartigen Prüfmaschine als Vereinzelungs- und Zuführeinrichtung fungieren.

Fig. 4 zeigt das Förderrad 110 von Fig. 2 nach Erweiterung mit einer durchsichtig dargestellten oberen Abdeckung 135, welche im Innenraum 121 parallel zum Radboden 111 angeordnet und nicht an der Rotation des Förderrades 110 teilzunehmen imstande ist. Beispielsweise kann die obere Abdeckung durch eine (nicht dargestellte) Halterung mit der Bodenplatte 150 verbunden und somit daran gehindert sein, an der Rotation teilzunehmen. Die obere Abdeckung 153 liegt entweder am Radboden 111 an oder ist in einer Distanz von diesem angeordnet, welche kleiner ist als der minimale Durchmesser der Objekte 6,7,8,9, und ist so angeordnet, dass sie als Abstreifer für solche im Innenraum 121 befindliche Objekte dient, welche noch nicht in eines der Löcher 112,112' hineingefallen sind, aber z.B. auf Grund von Adhäsion dennoch an der Rotation des Förderrades teilnehmen. Solche Objekte werden durch die obere Abdeckung 135 vom Radboden 111 abgestreift und fallen zurück in den Bereich des tiefsten Punktes des Innenraumes 121.

Insbesondere bei Verwendung einer derartigen oberen Abdeckung 135 kann die erfindungsgemäße Fördereinrichtung problemlos selbst bei senkrechter Stellung der Radachse Y betrieben werden.

Der Radboden 111 kann gegen einen solchen mit Löchern anderer Zahl oder von anderen Abmessungen auswechselbar sein, wodurch z.B. eine Anpassung der Fördereinrichtung an Objekte anderer Form oder Größe auf einfache Weise möglich ist.

Gemäß einer weiteren Variante gehen beide Aussparungen 131,132 ineinander über, d.h. sie stellen verschiedene Teile ein- und derselben Aussparung dar. Beispielsweise kann die untere Abdeckung 130 die untere Hälfte des Radbodens 111 abdecken, d.h. als halbe Kreisscheibe ausgebildet sein; die fehlende obere Hälfte der unteren Abdeckung fungiert dann als eine einzige große Aussparung, welche an die Stelle der Aussparungen 131,132 getreten ist.

Selbstverständlich können die Aussparungen 131,132 als Durchbrüche oder Löcher ausgebildet sein. Auch diese können ebenso ineinander übergehen, d.h. durch verschiedene Teile ein- und desselben Lochs bzw. Durchbruchs gebildet sein.

Somit besteht eine einfache Ausführungsform einer erfindungsgemäßen Fördervorrichtung aus einem drehbaren Becher bzw. Förderrad mit peripher angeordneten Mitnahmelöchern bzw. wenigstens einem Mitnahmeloch im Becherboden bzw. Radboden, deren Lochdurchmesser etwas größer ist als die zu fördernden Objekte (z.B. Tabletten oder Pillen oder Oblongs). Unterhalb des Becherbodens befindet sich gemäß dieser Ausführungsform eine feststehende Scheibe, deren Durchmesser entweder so groß oder etwas größer ist als der Durchmesser des Becherbodens. Diese Scheibe besitzt mindestens eine, vorzugsweise zwei, periphere Aussparungen oder Durchbrüche, welche vorzugsweise wenigstens etwas größer sind als die Mitnahmelöcher im Becherboden und welche mit den Mitnahmelöchern beim Überfahren koinzidieren, so dass beim Überfahren einer der Aussparungen durch ein Mitnahmeloch ein in dem Mitnahmeloch befindliches Objekt durch die Aussparung hindurch aus dem Becher herausfällt. Bei zwei Aussparungen innerhalb der Scheibe wird bei der Drehung des Bechers im Uhrzeigersinn die eine Aussparung, bei der Drehung im Gegenuhrzeigersinn die andere Aussparung als Auslass benutzt. Der Becher mitsamt der Scheibe kann gemäß einer schiefen Ebene geneigt sein oder auch waagrecht stehen, wobei die wirksamere Trennung von Objekten allerdings durch eine Schräglage des Bechers gegeben ist.

Eine Ausführung mit waagrechter Radachse des Bechers bzw. Förderrades und senkrecht stehenden Becherboden bzw. Radboden besitzt im Becherboden bzw. Radboden schräg nach außen gerichtet angeordnete Mitnahmelöcher. Eine feststehende Scheibe steht fest dem Becherboden gegenüber und besitzt unterhalb der Radachse eine oder auch zwei Aussparungen, welche wiederum, wie vorbeschrieben, mit den Mitnahmelöchern im Becherboden beim Überfahren koinzidieren und zusätzlich fluchten.

Gemäß einer Ausführungsform kann das Förderrad 110 mittels eines Vibrators wenigstens zeitweise in Vibration versetzt werden, um das Hineinfallen des Objekts in das Mitnahmeloch 112 bzw. in eines der Zusatz-Mitnahmelöcher 112' und danach den Auswurf des Objekts aus demselben sowie ferner das Sammeln der Übrigen Objekte im in tiefsten Bereich des Innenraums 121 zu unterstützen. Zu demselben Zweck kann alternativ oder zusätzlich die Rotation des Förderrades 110 intervallweise angehalten und wieder in Gang gesetzt werden.

Mit Hilfe einer erfindungsgemäßen Fördervorrichtung können Objekte ohne Verwendung einer Transportbahn vereinzelt werden. Die erfindungsgemäße Fördervorrichtung ermöglicht eine sehr schnelle, die Objekte schonende und zählgenaue Vereinzelung. Die ausgeworfenen und ggf. gezählten Objekte können in einem rückwärtig angeordneten Sammelbehälter aufgefangen werden. Eine Wägung der darin gesammelten Objekte zur Bestimmung ihrer Anzahl ist überflüssig. Bevorzugt sind alle reinigungsrelevanten Teile der Fördervorrichtung ohne Werkzeug demontierbar.

### Gewerbliche Anwendbarkeit:

Die Erfindung ist gewerblich anwendbar z.B. im Bereich der Pharmatechnik.

### Liste der Bezugszeichen:

- 6,7,8,9: Objekte, z.B. Tabletten
- 110: Förderrad
- 111: Radboden
- 112: Mitnahmeloch
- 112': Zusatz-Mitnahmeloch
- 114: Drehantrieb für 110
- 120: Seitenwandung von 110
- 121: Innenraum
- 130: untere Abdeckung
- 131, 132: Aussparungen in 130
- 135: obere Abdeckung
- 150: Fußplatte
- 151: Keil
- 152: Schlauch
- 161: erster Teil von PKR
- 162: zweiter Teil von PKR
- 163: dritter Teil von PKR
- 164: vierter Teil von PKR
- KR: Kreis der Bewegung von 112 um Y
- PKR: Projektion von KR auf die Ebene von 130 in Richtung von Y
- Y: Radachse von 110

## Patentansprüche

1. Fördervorrichtung für Objekte von fester oder gelartiger Konsistenz, insbesondere für pharmazeutische Objekte (6,7,8,9), wie Tabletten, Pillen oder Oblongs, mit einem drehbar gelagerten Förderrad (110), einem Drehantrieb (114) und einer unteren Abdeckung (130), wobei
- das Förderrad (110) durch einen Radboden (111) sowie eine den Innenraum (121) des Förderrades (110) peripher umschließende Seitenwandung (120) becher-, schüssel- oder muldenförmig ausgebildet ist, wobei der Innenraum (121) zur Aufnahme der Objekte (6,7,8,9) vor deren Vereinzelung dient,
- der Drehantrieb (114) imstande ist, das Förderrad (110) um dessen Radachse (Y) zu drehen,
- die Richtung der Radachse (Y) des Förderrades (110) einen vorgegebenen Kippwinkel (ψ) von 0° bis 90° zur Senkrechten einnimmt, wodurch sich die Objekte (9) im Bereich des tiefsten Punktes des Innenraumes (121) sammeln,
- die untere Abdeckung (130) auf der vom Innenraum (121) abgewandten Seite des Radbodens (111) parallel zu diesem angeordnet ist und nicht an der Rotation des Förderrades (110,110') teilzunehmen imstande ist,
- die untere Abdeckung (130) entweder am Radboden (111) anliegt oder in einer solchen Distanz von diesem angeordnet ist, welche kleiner ist als der minimale Durchmesser der Objekte (6,7,8,9),
- der Radboden (111) in einem vorgegebenen Abstand von der Radachse (Y) wenigstens ein Mitnahmeloch (112) aufweist, dessen Lage und Abmessungen so gewählt sind, dass genau eines der im Bereich des tiefsten Punktes des Innenraumes (121) versammelten Objekte (9) so in das Mitnahmeloch (112) hineinfallen kann, dass es in stabiler Lage am Rand des Mitnahmelochs (112) sowie auf der unteren Abdeckung (130) anliegt und **dadurch** imstande ist, unter Abrollen oder Gleiten auf der unteren Abdeckung (130) an der Rotation des Förderrades (110) teilzunehmen und auf diese Weise den Bereich des tiefsten Punktes des Innenraumes (121) zu verlassen, und
- die untere Abdeckung (130) einen ersten Teil (161) der zur Radachse (Y) parallelen Projektion (PKR) des Kreises (KR), welchen das Mitnahmeloch (112) um die Radachse (Y,Y') beschreibt, abdeckt, und einen zweiten Teil (162) dieser Projektion (PKR) nicht abdeckt, wodurch das in das Mitnahmeloch (112) hineingefallene Objekt (6,7) dann nach unten aus dem Mitnahmeloch (112) herausfällt und somit aus der Fördervorrichtung ausgeworfen wird, wenn die zur Radachse (Y) parallele Projektion des Mitnahmelochs (112) den zweiten Teil (162) erreicht,
so dass die Fördereinrichtung zur automatischen Vereinzelung der Objekte (6,7,8,9) imstande ist, **dadurch gekennzeichnet, dass**
- die Rotationsrichtung des Förderrades (110) umkehrbar ist, und dass
- die untere Abdeckung (130) auch einen vom zweiten Teil (162) beabstandeten dritten Teil (163) der zur Radachse (Y) parallelen Projektion des Kreises (KR), welchen das Mitnahmeloch (112) um die Radachse (Y) beschreibt, nicht abdeckt,
so dass das in das Mitnahmeloch (112) oder eines der Zusatz-Mitnahmelöcher (112') hineingefallene Objekt bei Rotation des Förderrades (110) in einer Richtung bei Erreichen des zweiten Teils (162) und bei Rotation des Förderrades (110) in der anderen Richtung bei Erreichen des dritten Teils (163) ausgeworfen wird und somit der Ort des Auswurfes durch Vorgeben der Rotationsrichtung des Förderrades (110) wählbar ist.

2. Fördervorrichtung nach Anspruch 1, **dadurch gekennzeichnet,**
**dass** sich der erste Teil über einen Kreisbogen erstreckt, welcher den Bereich des tiefsten Punktes der zur Radachse (Y) parallelen Projektion des Kreises (KR) umfaßt.

3. Fördervorrichtung nach einem der vorigen Ansprüche, **dadurch gekennzeichnet,**
**dass** sich der erste Teil wenigstens über einen Kreisbogen erstreckt, welcher sich vom tiefsten Punkt des Kreises (KR) aus über einen Winkel von wenigstens ±10°, vorzugsweise über einen Winkel von wenigstens ±30° erstreckt.

4. Fördervorrichtung nach einem der vorigen Ansprüche, **dadurch gekennzeichnet,**
**dass** die Radachse (Y) um einen Kippwinkel (ψ) zwischen 10° und 80°, vorzugsweise zwischen 30° und 60°, gegen die Senkrechte geneigt ist, so dass sich das Förderrad (110) in Schrägstellung befindet.

5. Fördervorrichtung nach Anspruch 4, **dadurch gekennzeichnet,**
**dass** der Kippwinkel (ψ) verstellbar ist.

6. Fördervorrichtung nach einem der vorigen Ansprüche, **dadurch gekennzeichnet,**
**dass** der Radboden (111) zusätzlich zu dem Mitnahmeloch (112) ein, mehrere oder viele Zusatz-Mitnahmelöcher (112') aufweist, deren Abmessungen mit denjenigen des Mitnahmelochs (112) übereinstimmen und welche gemeinsam mit dem Mitnahmeloch (112) alle auf einem Kreis (KR) liegen.

7. Fördervorrichtung nach einem der vorigen Ansprüche, **gekennzeichnet durch**
eine Auffangeinrichtung (152), insbesondere Schlauch (152), Rohr oder Rinne, welche die nach unten aus dem Mitnahmeloch (112) oder einem der Zusatz-Mitnahmelöcher (112') herausgefallenen Objekte zum Zweck der Weiterleitung aufnimmt.

8. Fördervorrichtung nach einem der vorigen Ansprüche, **dadurch gekennzeichnet,**
**dass** der Radboden (111) an seiner dem Innenraum zugewandten Seite konisch, kegelmantelfömig oder konvex ausgebildet ist.

9. Fördervorrichtung nach einem der vorigen Ansprüche, **gekennzeichnet durch**
einen Vibrator, welcher das Förderrad (110) wenigstens zeitweise in Vibration zu versetzen imstande ist.

10. Fördervorrichtung nach einem der vorigen Ansprüche, **dadurch gekennzeichnet,**
**dass** die Abmessungen des Mitnahmelochs (112) bzw. des Mitnahmelochs (112) und der Zusatz-Mitnahmelöcher (112') so gewählt sind, dass anstelle jeweils genau eines der Objekte jeweils eine vorgegebene Anzahl von genau n Objekten in das Mitnahmeloch (112) bzw. in das Mitnahmeloch (112) und die Zusatz-Mitnahmelöcher (112') hineinfallen und danach nach unten aus dem Mitnahmeloch (112) bzw. dem Zusatz-Mitnahmeloch (112') herausfallen können, so dass die Fördereinrichtung, anstatt zur automatischen Vereinzelung der Objekte imstande zu sein, zur automatischen Gruppierung der Objekte zu Gruppen von jeweils genau n Objekten imstande ist.

11. Fördervorrichtung nach einem der vorigen Ansprüche, **dadurch gekennzeichnet,**
**dass** die Rotationsgeschwindigkeit des Förderrades (110) veränderbar ist.

12. Fördervorrichtung nach einem der vorigen Ansprüche, **dadurch gekennzeichnet,**
**dass** die Rotation des Förderrades (110) intervallweise anhaltbar und wieder in Gang setzbar ist.

13. Fördervorrichtung nach einem der vorigen Ansprüche, **gekennzeichnet durch**
einen Aufprallsensor, mittels welchem die Zahl der ausgeworfenen Objekte zählbar ist.

14. Fördervorrichtung nach einem der vorigen Ansprüche, **gekennzeichnet durch**
eine obere Abdeckung (135), welche
- im Innenraum parallel zum Radboden (111) angeordnet und nicht an der Rotation des Förderrades (110) teilzunehmen imstande ist,
- entweder am Radboden (111) anliegt oder in einer solchen Distanz von diesem angeordnet ist, welche kleiner ist als der minimale Durchmesser der Objekte (6,7,8,9),
- und als Abstreifer für solche im Innenraum befindlichen Objekte (9) dient, welche nicht in das Mitnahmeloch (112) oder eines der Zusatz-Mitnahmelöcher (112') hineingefallen sind.

15. Fördervorrichtung nach einem der vorigen Ansprüche, **dadurch gekennzeichnet,**
**dass** der Drehantrieb (114) imstande ist, das Förderrad durch mehrmaliges aufeinanderfolgendes Umschalten der Rotationsrichtung in eine Schaukelbewegung zu versetzen.

16. Fördervorrichtung nach einem der vorigen Ansprüche, **dadurch gekennzeichnet,**
**dass** der Radboden (111) auswechselbar ist gegen einen solchen mit
- einem Mitnahmeloch von anderen Abmessungen oder,
- Mitnahmelöchern bzw. Zusatz-Mitnahmelöchern von anderen Abmessungen, oder
- Mitnahmelöchern bzw. Zusatz-Mitnahmelöchern von anderer Zahl.

17. Verfahren zur Förderung von Objekten von fester oder gelartiger Konsistenz, insbesondere von pharmazeutischen Objekten (6,7,8,9), wie Tabletten, Pillen oder Oblongs, unter Verwendung eines drehbar gelagerten Förderrades (110), eines Drehantriebes (114) und einer unteren Abdeckung (130), wobei
- als Förderrad (110) ein solches verwendet wird, welches durch einen Radboden (111) sowie eine den Innenraum (121) des Förderrades (110) peripher umschließende Seitenwandung (120) becher-, schüssel- oder muldenförmig ausgebildet ist, wobei der Innenraum (121) zur Aufnahme der Objekte (6,7,8,9) vor deren Vereinzelung dient,
- das Förderrad durch den Drehantrieb um die Radachse (Y) des Förderrades (110) gedreht wird, wobei die Richtung der Radachse (Y) des Förderrades (110) um einen vogegebenen Kippwinkel (ψ) von 0° bis 90° zur Senkrechten einnimmt, wodurch sich die Objekte (9) im Bereich des tiefsten Punktes des Innenraumes (121) sammeln,
- als untere Abdeckung (130) eine solche verwendet wird, welche auf der vom Innenraum (121) abgewandten Seite des Radbodens (111) parallel zu diesem angeordnet ist und nicht an der Rotation des Förderrades (110) teilzunehmen imstande ist, und welche entweder am Radboden (111) anliegt oder in einer solchen Distanz von diesem angeordnet ist, welche kleiner ist als der minimale Durchmesser der Objekte (6,7,8,9),
- als Radboden (111) ein solcher verwendet wird, welcher in einem vorgegebenen Abstand von der Radachse (Y) wenigstens ein Mitnahmeloch (112) aufweist, dessen Lage und Abmessungen so gewählt sind, dass genau eines der im Bereich des tiefsten Punktes des Innenraumes (121) versammelten Objekte (9) so in das Mitnahmeloch (112) hineinfallen kann, dass es in stabiler Lage am Rand des Mitnahmelochs (112) sowie auf der unteren Abdeckung (130) anliegt und **dadurch** imstande ist, unter Abrollen oder Gleiten auf der unteren Abdeckung (130) an der Rotation des Förderrades (110) teilzunehmen und auf diese Weise den Bereich des tiefsten Punktes des Innenraumes (121) zu verlassen, und
- als untere Abdeckung (130) eine solche verwendet wird, welche einen ersten Teil (161) der zur Radachse (Y) parallelen Projektion (PKR) des Kreises (KR), welchen das Mitnahmeloch (112) um die Radachse (Y) beschreibt, abdeckt, und einen zweiten Teil (162) dieser Projektion (PKR) nicht abdeckt, wodurch das in das Mitnahmeloch (112) hineingefallene Objekt (6,7) dann nach unten aus dem Mitnahmeloch (112) herausfällt und somit aus der Fördervorrichtung ausgeworfen wird, wenn die zur Radachse (Y) parallele Projektion des Mitnahmelochs (112) den zweiten Teil (162) erreicht,
so dass mittels der Fördereinrichtung die Objekte (6,7,8,9) automatisch vereinzelt werden, **dadurch gekennzeichnet, dass**
- die Rotationsrichtung des Förderrades (110) in bestimmten regelmäßigen oder unregelmäßigen Zeitabständen umgekehrt wird, und dass
- als untere Abdeckung (130) eine solche verwendet wird, welche auch einen vom zweiten Teil (162) beabstandeten dritten Teil (163) der zur Radachse (Y) parallelen Projektion des Kreises (KR), welchen das Mitnahmeloch (112) um die Radachse (Y) beschreibt, nicht abdeckt,
so dass das in das Mitnahmeloch (112) oder eines der Zusatz-Mitnahmelöcher (112') hineingefallene Objekt bei Rotation des Förderrades (110) in einer Richtung bei Erreichen des zweiten Teils (162) und bei Rotation des Förderrades (110) in der anderen Richtung bei Erreichen des dritten Teils (163) ausgeworfen wird und somit der Ort des Auswurfes durch Vorgeben der Rotationsrichtung des Förderrades (110) wählbar ist.

18. Verfahren nach Anspruch 17, **dadurch gekennzeichnet,**
**dass** sich der erste Teil über einen Kreisbogen erstreckt, welcher den Bereich des tiefsten Punktes der zur Radachse (Y) parallelen Projektion des Kreises (KR) umfaßt.

19. Verfahren nach Anspruch 17 oder 18, **dadurch gekennzeichnet,**
**dass** sich der erste Teil wenigstens über einen Kreisbogen erstreckt, welcher sich vom tiefsten Punkt des Kreises (KR) aus über einen Winkel von wenigstens ±10°, vorzugsweise über einen Winkel von wenigstens ±30° erstreckt.

20. Verfahren nach einem der Ansprüche 17 bis 19, **dadurch gekennzeichnet,**
**dass** die Radachse (Y) um einen Kippwinkel (ψ) zwischen 10° und 80°, vorzugsweise zwischen 30° und 60°, gegen die Senkrechte geneigt ist, so dass sich das Förderrad (110) in Schrägstellung befindet.

21. Verfahren nach Anspruch 20, **dadurch gekennzeichnet,**
**dass** der Kippwinkel (ψ) verstellbar ist.

22. Verfahren nach einem der Ansprüche 17 bis 21, **dadurch gekennzeichnet,**
**dass** als Radboden ein solcher verwendet wird, welcher zusätzlich zu dem Mitnahmeloch (112) ein, mehrere oder viele Zusatz-Mitnahmelöcher (112') aufweist, deren Abmessungen mit denjenigen des Mitnahmeochs (112) übereinstimmen und welche gemeinsam mit dem Mitnahmeloch (112) alle auf einem Kreis (KR) liegen.

23. Verfahren nach einem der Ansprüche 17 bis 22, **dadurch gekennzeichnet,**
**dass** eine Auffangeinrichtung (152), insbesondere Schlauch (152), Rohr oder Rinne, verwendet wird, welche die nach unten aus dem Mitnahmeloch (112) oder einem der Zusatz-Mitnahmelöcher (112') herausgefallenen Objekte zum Zweck der Weiterleitung aufnimmt.

24. Verfahren nach einem der Ansprüche 17 bis 23, **dadurch gekennzeichnet,**
**dass** der Radboden (111) an seiner dem Innenraum zugewandten Seite konisch, kegelmantelfömig oder konvex ausgebildet ist.

25. Verfahren nach einem der Ansprüche 17 bis 24, **dadurch gekennzeichnet,**
**dass** das Förderrad (110) wenigstens zeitweise durch einen Vibrator in Vibration versetzt wird.

26. Verfahren nach einem der Ansprüche 17 bis 25, **dadurch gekennzeichnet,**
**dass** die Abmessungen des Mitnahmelochs (112) bzw. des Mitnahmelochs (112) und der Zusatz-Mitnahmelöcher (112') so gewählt sind, dass anstelle jeweils genau eines der Objekte jeweils eine vorgegebene Anzahl von genau n Objekten in das Mitnahmeloch (112) bzw. in das Mitnahmeloch (112) und die Zusatz-Mitnahmelöcher (112') hineinfallen und danach nach unten aus dem Mitnahmeloch (112) bzw. dem Zusatz-Mitnahmeloch (112') herausfallen können, so dass die Fördereinrichtung, anstatt zur automatischen Vereinzelung der Objekte imstande zu sein, zur automatischen Gruppierung der Objekte zu Gruppen von jeweils genau n Objekten imstande ist.

27. Verfahren nach einem der Ansprüche 17 bis 26, **dadurch gekennzeichnet,**
**dass** die Rotationsgeschwindigkeit des Förderrades (110) verändert wird.

28. Verfahren nach einem der Ansprüche 17 bis 27, **dadurch gekennzeichnet,**
**dass** die Rotation des Förderrades (110) intervallweise angehalten und wieder in Gang gesetzt wird.

29. Verfahren nach einem der Ansprüche 17 bis 28, **dadurch gekennzeichnet,**
**dass** die Zahl der ausgeworfenen Objekte (8) mittels eines Aufprallsensors gezählt wird.

30. Verfahren nach einem der Ansprüche 17 bis 29, **dadurch gekennzeichnet,**
**dass** eine obere Abdeckung (135) verwendet wird, welche
- im Innenraum parallel zum Radboden (111) angeordnet und nicht an der Rotation des Förderrades (110) teilzunehmen imstande ist,
- entweder am Radboden (111) anliegt oder in einer solchen Distanz von diesem angeordnet ist, welche kleiner ist als der minimale Durchmesser der Objekte (6,7,8,9),
- und als Abstreifer für solche im Innenraum befindlichen Objekte (9) dient, welche nicht in das Mitnahmeloch (112) oder eines der Zusatz-Mitnahmelöcher (112') hineingefallen sind.

31. Verfahren nach einem der Ansprüche 17 bis 30, **dadurch gekennzeichnet,**
**dass** als Drehantrieb (114) ein solcher verwendet wird, welcher imstande ist, das Förderrad durch mehrmaliges aufeinanderfolgendes Umschalten der Rotationsrichtung in eine Schaukelbewegung zu versetzen.

32. Verfahren nach einem der Ansprüche 17 bis 31, **dadurch gekennzeichnet,**
**dass** als Radboden (111) ein solcher verwendet wird, welcher auswechselbar ist gegen einen solchen mit
- einem Mitnahmeloch von anderen Abmessungen oder,
- Mitnahmelöchern bzw. Zusatz-Mitnahmelöchern von anderen Abmessungen, oder
- Mitnahmelöchern bzw. Zusatz-Mitnahmelöchern von anderer Zahl.

## Claims

1. A conveying device for objects having a solid or gel-like consistency, especially for pharmaceutical objects (6, 7, 8, 9) such as tablets, pills or capsules, comprising a rotatably mounted conveying wheel (110), a rotary drive (114) and a bottom cover (130), whereby
• the conveying wheel (110) consists of a wheel base (111) as well as of a side wall (120) that peripherally surrounds the inner chamber (121) of the conveying wheel (110) in such a way that it has a cup-shaped, bowl-shaped or trough-shaped configuration, whereby the inner chamber (121) serves to receive the objects (6, 7, 8, 9) before they are individuated,
• the rotary drive (114) is capable of turning the conveying wheel (110) around its axis (Y),
• the direction of the axis (Y) of the conveying wheel (110) assumes a prescribed tilting angle (Ψ) of 0° to 90° relative to the perpendicular, as a result of which the objects (9) collect in the area of the lowest point of the inner chamber (121),
• the bottom cover (130) is arranged on the side of the wheel base (111) facing away from the inner chamber (121) and parallel thereto and it is not capable of participating in the rotation of the conveying wheel (110),
• the bottom cover (130) either rests on the wheel base (111) or is arranged at a distance from it that is smaller than the minimum diameter of the objects (6, 7, 8, 9),
• at a prescribed distance from the wheel axis (Y), the wheel base (111) has at least one catching hole (112) whose positioning and dimensions are selected in such a way that precisely one of the objects (9) collected in the area of the lowest point of the inner chamber (121) can fall into the catching hole (112) in such a way that it rests in a stable position on the edge of the catching hole (112) as well as on the bottom cover (130), as a result of which, while said object (9) rolls or slides on the bottom cover (130), it is capable of participating in the rotation of the conveying wheel (110) and thus of leaving the area of the lowest point of the inner chamber (121), and
• the bottom cover (130) covers a first part (161) of the projection (PKR) - parallel to the wheel axis (Y) - of the circle (KR) described around the wheel axis (Y) by the catching hole (112), and said bottom cover (130) does not cover a second part (162) of this projection (PKR), as a result of which the object (6, 7) that has fallen into the catching hole (112) then falls downwards out of the catching hole (112) and is thus ejected from the conveying device when the projection of the catching hole (112) that is parallel to the wheel axis (Y) reaches the second part (162),
so that the conveying device is capable of automatically individuating the objects (6, 7, 8, 9), **characterized in that**
• the direction of rotation of the conveying wheel (110) can be reversed and **in that**
• the bottom cover (130) likewise does not cover a third part (163) of the projection - parallel to the wheel axis (Y) - of the circle (KR) described around the wheel axis (Y) by the catching hole (112), said third part (163) being at a distance from the second part (162),
so that the object that has fallen into the catching hole (112) or into one of the additional catching holes (112') is ejected when it reaches the second part (162) as the conveying wheel (110) rotates in one direction and when it reaches the third part (163) as the conveying wheel (110) rotates in the other direction, and consequently, the site of the ejection can be selected by prescribing the direction of rotation of the conveying wheel (110).

2. The conveying device according to Claim 1, **characterized in that**
the first part extends over a circle arc that encompasses the area of the lowest point of the projection - parallel to the wheel axis (Y) - of the circle (KR).

3. The conveying device according to any of the preceding claims, **characterized in that**
the first part extends at least over a circle arc that extends from the lowest point of the circle (KR) over an angle of at least ± 10°, preferably over an angle of at least ± 30°.

4. The conveying device according to any of the preceding claims, **characterized in that**
the wheel axis (Y) is slanted by a tilting angle (Ψ) between 10° and 80°, preferably between 30° and 60°, relative to the perpendicular, so that the conveying wheel (110) is in a slanted position.

5. The conveying device according to Claim 4, **characterized in that**
the tilting angle (Ψ) is adjustable.

6. The conveying device according to any of the preceding claims, **characterized in that**
the wheel base (111), in addition to the catching hole (112), has one, several or many additional catching holes (112') whose dimensions correspond to those of the catching hole (112) and which all lie on a circle (KR) together with the catching hole (112).

7. The conveying device according to any of the preceding claims, **characterized by**
a collecting device (152), especially a tube (152), pipe or trough, that receives the objects that have fallen downwards out of the catching hole (112) or out of one of the additional catching holes (112'), for purposes of further conveyance.

8. The conveying device according to any of the preceding claims, **characterized in that**
the side of the wheel base (111) facing the inner chamber is configured to be conical, convex or like the envelope of a cone.

9. The conveying device according to any of the preceding claims, **characterized by**
a vibrator that is capable of making the conveying wheel (110) vibrate, at least temporarily.

10. The conveying device according to any of the preceding claims, **characterized in that**
the dimensions of the catching hole (112) or of the catching hole (112) and of the additional catching holes (112') are selected in such a way that, instead of precisely one of the objects, in each case, a prescribed number of precisely n objects falls into the catching hole (112) or into the catching hole (112) and the additional catching holes (112') and can then fall downwards out of the catching hole (112) or out of the additional catching holes (112'), so that the conveying device, instead of being capable of automatically individuating the objects, is capable of automatically grouping the objects into groups of precisely n objects in each case.

11. The conveying device according to any of the preceding claims, **characterized in that**
the rotational speed of the conveying wheel (110) is variable.

12. The conveying device according to any of the preceding claims, **characterized in that**
the rotation of the conveying wheel (110) can be stopped and resumed at given intervals.

13. The conveying device according to any of the preceding claims, **characterized by**
an impact sensor by means of which the number of ejected objects can be counted.

14. The conveying device according to any of the preceding claims, **characterized by** a top cover (135) that
• is arranged in the inner chamber parallel to the wheel base (111) and that is not capable of participating in the rotation of the conveying wheel (110),
• either rests on the wheel base (111) or is arranged at a distance from it that is smaller than the minimum diameter of the objects (6, 7, 8, 9),
• and serves as a scraper for objects (9) located in the inner chamber that have not fallen into the catching hole (112) or into one of the additional catching holes (112').

15. The conveying device according to any of the preceding claims, **characterized in that**
the rotary drive (114) is capable of imparting a pendulum movement to the conveying wheel by successively reversing the direction of rotation several times.

16. The conveying device according to any of the preceding claims, **characterized in that**
the wheel base (111) can be replaced with one having
• one catching hole of different dimensions,
• catching holes or additional catching holes of different dimensions, or
• a different number of catching holes or additional catching holes.

17. A method for conveying objects having a solid or gel-like consistency, especially for pharmaceutical objects (6, 7, 8, 9) such as tablets, pills or capsules, making use of a rotatably mounted conveying wheel (110), a rotary drive (114) and a bottom cover (130), whereby
• the conveying wheel (110) employed is one that consists of a wheel base (111) as well as of a side wall (120) that peripherally surrounds the inner chamber (121) of the conveying wheel (110) in such a way that it has a cup-shaped, bowl-shaped or trough-shaped configuration, whereby the inner chamber (121) serves to receive the objects (6, 7, 8, 9) before they are individuated,
• the conveying wheel is turned by the rotary drive around the axis (Y) of the conveying wheel (110), whereby the direction of the axis (Y) of the conveying wheel (110) assumes a prescribed tilting angle (Ψ) of 0° to 90° relative to the perpendicular, as a result of which the objects (9) collect in the area of the lowest point of the inner chamber (121),
• the bottom cover (130) employed is one that is arranged on the side of the wheel base (111) facing away from the inner chamber (121) and parallel thereto and that is not capable of participating in the rotation of the conveying wheel (110), and that either rests on the wheel base (111) or is arranged at a distance from it that is smaller than the minimum diameter of the objects (6, 7, 8, 9),
• the wheel base (111) employed is one that, at a prescribed distance from the wheel axis (Y), has at least one catching hole (112) whose positioning and dimensions are selected in such a way that precisely one of the objects (9) collected in the area of the lowest point of the inner chamber (121) can fall into the catching hole (112) in such a way that it rests in a stable position on the edge of the catching hole (112) as well as on the bottom cover (130), as a result of which, while said object (9) rolls or slides on the bottom cover (130), it is capable of participating in the rotation of the conveying wheel (110) and thus of leaving the area of the lowest point of the inner chamber (121), and
• the bottom cover (130) employed is one that covers a first part (161) of the projection (PKR) - parallel to the wheel axis (Y) - of the circle (KR) described around the wheel axis (Y) by the catching hole (112), and said bottom cover (130) does not cover a second part (162) of this projection (PKR), as a result of which the object (6, 7) that has fallen into the catching hole (112) then falls downwards out of the catching hole (112) and is thus ejected from the conveying device when the projection of the catching hole (112) that is parallel to the wheel axis (Y) reaches the second part (162),
so that the objects (6, 7, 8, 9) are automatically individuated by means of the conveying device, **characterized in that**
• the direction of rotation of the conveying wheel (110) can be reversed at certain regular or irregular time intervals and **in that**
• the bottom cover (130) employed is one that likewise does not cover a third part (163) of the projection - parallel to the wheel axis (Y) - of the circle (KR) described around the wheel axis (Y) by the catching hole (112), said third part (163) being at a distance from the second part (162),
so that the object that has fallen into the catching hole (112) or into one of the additional catching holes (112') is ejected when it reaches the second part (162) as the conveying wheel (110) rotates in one direction and when it reaches the third part (163) as the conveying wheel (110) rotates in the other direction, and consequently, the site of the ejection can be selected by prescribing the direction of rotation of the conveying wheel (110).

18. The method according to Claim 17, **characterized in that**
the first part extends over a circle arc that encompasses the area of the lowest point of the projection - parallel to the wheel axis (Y) - of the circle (KR).

19. The method according to Claim 17 or 18, **characterized in that**
the first part extends at least over a circle arc that extends from the lowest point of the circle (KR) over an angle of at least ± 10°, preferably over an angle of at least ± 30°.

20. The method according to any of Claims 17 to 19, **characterized in that**
the wheel axis (Y) is slanted by a tilting angle (Ψ) between 10° and 80°, preferably between 30° and 60°, relative to the perpendicular, so that the conveying wheel (110) is in a slanted position.

21. The method according to Claim 20, **characterized in that**
the tilting angle (Ψ) is adjustable.

22. The method according to any of Claims 17 to 21, **characterized in that**
the wheel base (111) employed is one that, in addition to the catching hole (112), has one, several or many additional catching holes. (112') whose dimensions correspond to those of the catching hole (112) and which all lie on a circle (KR) together with the catching hole (112).

23. The method according to any of Claims 17 to 22, **characterized in that**
a collecting device (152), especially a tube (152), pipe or trough is employed that receives the objects that have fallen downwards out of the catching hole (112) or out of one of the additional catching holes (112'), for purposes of further conveyance.

24. The method according to any of Claims 17 to 23, **characterized in that**
the side of the wheel base (111) facing the inner chamber is configured to be conical, convex or like the envelope of a cone.

25. The method according to any of Claims 17 to 24, **characterized in that**
the conveying wheel (110) is made to vibrate, at least temporarily, by a vibrator.

26. The method according to any of Claims 17 to 25, **characterized in that**
the dimensions of the catching hole (112) or of the catching hole (112) and of the additional catching holes (112') are selected in such a way that, instead of precisely one of the objects, in each case, a prescribed number of precisely n objects falls into the catching hole (112) or into the catching hole (112) and the additional catching holes (112') and can then fall downwards out of the catching hole (112) or out of the additional catching holes (112'), so that the conveying device, instead of being capable of automatically individuating the objects, is capable of automatically grouping the objects into groups of precisely n objects in each case.

27. The method according to any of Claims 17 to 26, **characterized in that**
the rotational speed of the conveying wheel (110) is varied.

28. The method according to any of Claims 17 to 27, **characterized in that**
the rotation of the conveying wheel (110) is stopped and resumed at given intervals.

29. The method according to any of Claims 17 to 28, **characterized in that**
the number of ejected objects (8) is counted by means of an impact sensor.

30. The method according to any of Claims 17 to 29, **characterized in that** a top cover (135) is employed that
• is arranged in the inner chamber parallel to the wheel base (111) and that is not capable of participating in the rotation of the conveying wheel (110),
• either rests on the wheel base (111) or is arranged at a distance from it that is smaller than the minimum diameter of the objects (6, 7, 8, 9),
• and serves as a scraper for objects (9) located in the inner chamber that have not fallen into the catching hole (112) or into one of the additional catching holes (112').

31. The method according to any of Claims 17 to 30, **characterized in that**
the rotary drive (114) employed is one that is capable of imparting a pendulum movement to the conveying wheel by successively reversing the direction of rotation several times.

32. The method according to any of Claims 17 to 30, **characterized in that**
the wheel base (111) employed is one that can be replaced with one having
• one catching hole of different dimensions,
• catching holes or additional catching holes of different dimensions, or
• a different number of catching holes or additional catching holes.

## Revendications

1. Dispositif convoyeur pour objets de consistance solide ou de consistance de gélule, en particulier pour objets pharmaceutiques (6,7,8,9), tels que des comprimés, des pilules ou des oblongs, comprenant une roue convoyeuse (110) tournant sur palier, un moteur d'orientation (114) et un recouvrement inférieur (130), dans lequel
- la roue convoyeuse (110) est formée par un fond de la roue (111), ainsi que par une paroi latérale (120) en forme de coupe, de cuve ou d'auge entourant l'espace intérieur (121) de la roue convoyeuse (110) en sa périphérie, l'espace intérieur (121) servant à la réception des objets (6,7,8,9) avant leur séparation,
- le moteur d'orientation (114) est en mesure de faire tourner la roue convoyeuse (110) autour de l'axe de la roue (Y) de celle-ci,
- l'orientation de l'axe de la roue (Y) de la roue convoyeuse (110) prend un angle d'inclinaison (ψ) prédéfini de 0° à 90° par rapport à la verticale, ce par quoi les objets (9) s'amassent dans la zone du point le plus profond de l'espace intérieur (121),
- le recouvrement inférieur (130) est disposé sur le côté du fond de la roue (111) opposé à l'espace intérieur (121), parallèlement au fond de la roue et n'est pas en mesure de participer à la rotation de la roue convoyeuse (110),
- le recouvrement inférieur (130) est disposé ou bien apposé au fond de la roue (111) ou bien à une distance telle de celui-ci qu'elle est plus petite que le diamètre minima des objets (6,7,8,9),
- le fond de la roue (111) présente à une distance prédéfinie de l'axe de la roue (Y) au moins un trou d'entraînement (112) dont la position et les dimensions sont choisies de sorte que exactement l'un des objets (9) amassés dans la zone du point le plus profond de l'espace intérieur (121) peut tomber dans le trou d'entraînement (112), de sorte qu'il est appliqué dans une position stable contre le bord du trou d'entraînement (112), ainsi que sur le recouvrement inférieur (130) et est de ce fait en mesure de participer, par roulement ou glissement sur le recouvrement inférieur (130), à la rotation de la roue convoyeuse (110) et de quitter de cette façon la zone du point le plus profond de l'espace inférieur (121), et
- le recouvrement inférieur (130) recouvre une première partie (161) de la projection (PKR) parallèle à l'axe de la roue (Y) du cercle (KR) que le trou d'entraînement (112) décrit autour de l'axe de la roue (Y) et ne recouvre pas une deuxième partie (162) de cette projection (PKR), ce par quoi l'objet (6,7) tombé dans le trou d'entraînement (112) tombe ensuite vers le bas hors du trou d'entraînement (112) et est de ce fait éjecté hors du dispositif convoyeur lorsque la projection du trou d'entraînement (112) parallèle à l'axe de la roue (Y) atteint la deuxième partie (162),
de sorte que le dispositif convoyeur est en mesure de séparer automatiquement les objets (6,7,8,9), **caractérisé en ce que**
- le sens de rotation de la roue convoyeuse (110) est inversible, et que
- le recouvrement inférieur (130) ne recouvre pas non plus une troisième partie (163) espacée de la deuxième partie (162) de la projection parallèle à l'axe de la roue (Y) du cercle (KR) que le trou d'entraînement (112) décrit autour de l'axe de la roue (Y),
de sorte que l'objet tombé dans le trou d'entraînement (112) ou dans l'un des trous d'entraînement supplémentaires (112') est éjecté lors de la rotation de la roue convoyeuse (110) dans un sens lorsqu'il atteint la deuxième partie (162) et lors de la rotation de la roue convoyeuse (110) dans l'autre sens lorsqu'il atteint la troisième partie (163) et que de ce fait, l'endroit de l'éjection peut être choisi par la détermination du sens de rotation de la roue convoyeuse (110).

2. Dispositif convoyeur selon la revendication 1, **caractérisé en ce que**
la première partie s'étend sur un arc de cercle qui comprend la zone du point le plus profond de la projection du cercle (KR) parallèle à l'axe de la roue (Y).

3. Dispositif convoyeur selon l'une des revendications précédentes, **caractérisé en ce que** la première partie s'étend au moins sur un arc de cercle qui s'étend depuis le point le plus profond du cercle (KR) sur un angle de au moins ± 10°, de préférence sur un angle de ± 30°.

4. Dispositif convoyeur selon l'une des revendications précédentes, **caractérisé en ce que**
l'axe de la roue (Y) est incliné par rapport à la verticale d'un angle d'inclinaison (ψ) compris entre 10° et 80°, de préférence entre 30° et 60°, de sorte que la roue convoyeuse (110) se trouve à l'oblique.

5. Dispositif convoyeur selon la revendication 4, **carctérisé en ce que**
l'angle d'inclinaison (ψ) est réglable.

6. Dispositif convoyeur selon l'une des revendications précédentes, **caractérisé en ce que**
le fond de la roue (111) présente en plus du trou d'entraînement (112), un, plusieurs, ou de nombreux trous d'entraînement supplémentaires (112') dont les dimensions concordent avec celles du trou d'entraînement (112) et qui se trouvent tous avec le trou d'entraînement (112) sur un même cercle (KR).

7. Dispositif convoyeur selon l'une des revendications précédentes, **caractérisé par** un dispositif collecteur (152), notamment un tuyau flexible (152), un tube ou un conduit, lequel recueille les objets tombés vers le bas hors du trou d'entraînement (112) ou de l'un des trous d'entraînement supplémentaires (112'), à des fins d'acheminement ultérieur.

8. Dispositif convoyeur selon l'une des revendications précédentes, **caractérisé en ce que**
le fond de la roue (111) est conformé sur son côté tourné vers l'espace intérieur, de façon conoïde, conique ou convexe.

9. Dispositif convoyeur selon l'une des revendications précédentes, **caractérisé par** un vibrateur qui est en mesure de faire vibrer la roue convoyeuse (110), tout au moins temporairement.

10. Dispositif convoyeur selon l'une des revendications précédentes, **caractérisé en ce que**
les dimensions du trou d'entraînement (112), respectivement du trou d'entraînement (112) et des trous d'entraînement supplémentaires (112') sont choisies de sorte que, au lieu que ce soit à chaque fois exactement l'un des objets, ce sont à chaque fois un nombre prédéfini d'exactement « n » objets qui tombent dans le trou d'entraînement (112), respectivement dans le trou d'entraînement (112) et dans les trous d'entraînement supplémentaires (112'), et peuvent ensuite tomber hors du trou d'entraînement (112), respectivement hors du trou d'entraînement supplémentaire (112'), de sorte que, au lieu d'être en mesure de séparer automatiquement les objets, le dispositif convoyeur est en mesure de regrouper automatiquement les objets en respectivement exactement « n » objets.

11. Dispositif convoyeur selon l'une des revendications précédentes, **caractérisé en ce que**
la vitesse de rotation de la roue convoyeuse (110) est variable.

12. Dispositif convoyeur selon l'une des revendications précédentes, **caractérisé en ce que**
la rotation de la roue convoyeuse (110) peut être arrêtée par intervalles et remise en marche.

13. Dispositif convoyeur selon l'une des revendications précédentes, **caractérisé par** un capteur de chocs au moyen duquel le nombre des objets éjectés peut être compté.

14. Dispositif convoyeur selon l'une des revendications précédentes, **caractérisé par** un recouvrement supérieur (135), lequel
- est disposé dans l'espace intérieur parallèlement au fond de la roue (111) et n'est pas en mesure de participer à la rotation de la roue convoyeuse (110),
- est ou bien apposé au fond de la roue (111), ou bien disposé à une distance telle de celui-ci qu'elle est plus petite que le diamètre minima des objets (6,7,8,9),
- sert aussi de râcle pour les objets (9) se trouvant dans l'espace intérieur, et qui ne sont pas tombés dans le trou d'entraînement (112) ou l'un des trous supplémentaires (112').

15. Dispositif convoyeur selon l'une des revendications précédentes, **caractérisé en ce que**
le moteur d'orientation (114) est en mesure de conférer à la roue convoyeuse un mouvement de bascule par la commutation du sens de rotation réitérée successivement plusieurs fois.

16. Dispositif convoyeur selon l'une des revendications précédentes, **caractérisé en ce que**
le fond de la roue (111) est interchangeable contre un fond de roue avec
- un trou d'entraînement ayant d'autres dimensions, ou
- des trous d'entraînement, respectivement des trous d'entraînement supplémentaires ayant d'autres dimensions, ou
- des trous d'entraînement, respectivement des trous d'entraînement supplémentaires en nombre différent.

17. Procédé de convoyage d'objets de consistance solide ou de consistance de gélule, en particulier d'objets pharmaceutiques (6,7,8,9), tels que des comprimés, des pilules ou des oblongs, ayant recours à une roue convoyeuse tournant sur palier (110), un moteur d'orientation (114) et à un recouvrement inférieur (130), avec lequel
- la roue convoyeuse (110) utilisée est formée par un fond de la roue (111), ainsi que par une paroi latérale (120) en forme de coupe, de cuve ou d'auge entourant l'espace intérieur (121) de la roue convoyeuse (110) en sa périphérie, l'espace intérieur (121) servant à la réception des objets (6,7,8,9) avant leur séparation,
- le moteur d'orientation fait tourner la roue convoyeuse autour de l'axe de la roue (Y) de la roue convoyeuse (110), l'orientation de l'axe de la roue (Y) de la roue convoyeuse (110) prenant un angle d'inclinaison (ψ) prédéfini de 0° à 90° par rapport à la verticale, ce par quoi les objets (9) s'amassent dans la zone du point le plus profond de l'espace intérieur (121),
- le recouvrement inférieur (130) utilisé est disposé sur le côté du fond de la roue (111) opposé à l'espace intérieur (121), parallèlement au fond de la roue (111) et n'est pas en mesure de participer à la rotation de la roue convoyeuse (110), et est disposé ou bien apposé au fond de la roue (111), ou bien à une distance telle de celui-ci qu'elle est plus petite que le diamètre minima des objets (6,7,8,9),
- le fond de la roue (111) utilisé présente à une distance prédéfinie de l'axe de la roue (Y) au moins un trou d'entraînement (112) dont la position et les dimensions sont choisies de sorte que exactement l'un des objets (9) amassés dans la zone du point le plus profond de l'espace intérieur (121) peut tomber dans le trou d'entraînement (112), de sorte qu'il est appliqué dans une position stable contre le bord du trou d'entraînement (112), ainsi que sur le recouvrement inférieur (130) et est de ce fait en mesure de participer par roulement ou glissement sur le recouvrement inférieur (130) à la rotation de la roue convoyeuse (110) et de quitter de cette façon la zone du point le plus profond de l'espace intérieur (121), et
- le recouvrement inférieur (130) utilisé recouvre une première partie (161) de la projection (PKR) parallèle à l'axe de la roue (Y) du cercle (KR) que le trou d'entraînement (112) décrit autour de l'axe de la roue (Y) et ne recouvre pas une deuxième partie (162) de cette projection (PKR), ce par quoi l'objet (6,7) tombé dans le trou d'entraînement (112) tombe ensuite vers le bas hors du trou d'entraînement (112) et est de ce fait éjecté hors du dispositif convoyeur lorsque la projection du trou d'entraînement (112) parallèle à l'axe de la roue (Y) atteint la deuxième partie (162),
de sorte que les objets (6,7,8,9) peuvent être séparés automatiquement au moyen du dispositif convoyeur, **caractérisé en ce que**
- le sens de rotation de la roue convoyeuse (110) peut être inversé à des espaces dans le temps définis, réguliers ou irréguliers, et que
- le recouvrement inférieur (130) utilisé ne recouvre pas non plus une troisième partie (163) espacée de la deuxième partie (162) de la projection parallèle à l'axe de la roue (Y) du cercle (KR) que le trou d'entraînement (112) décrit autour de l'axe de la roue (Y),
de sorte que l'objet tombé dans le trou d'entraînement (112) ou dans l'un des trous d'entraînement supplémentaires (112') est éjecté lors de la rotation de la roue convoyeuse (110) dans un sens lorsqu'il atteint la deuxième partie (162) et lors de la rotation de la roue convoyeuse (110) dans l'autre sens lorsqu'il atteint la troisième partie (163) et que de ce fait, l'endroit de l'éjection peut être choisi par la détermination du sens de rotation de la roue convoyeuse (110).

18. Procédé selon la revendication 17, **caractérisé en ce que**
la première partie s'étend sur un arc de cercle qui comprend la zone du point le plus profond de la projection du cercle (KR) parallèle à l'axe de la roue (Y).

19. Dispositif selon la revendication 17 ou 18, **caractérisé en ce que**
la première partie s'étend sur au moins un arc de cercle qui s'étend depuis le point le plus profond du cercle (KR) sur un angle de au moins ± 10°, de préférence sur un angle de ± 30°.

20. Procédé selon l'une des revendications 17 à 19, **caractérisé en ce que**
l'axe de la roue (Y) est incliné par rapport à la verticale d'un angle d'inclinaison (ψ) compris entre 10° et 80°, de préférence entre 30° et 60°, de sorte que la roue convoyeuse (110) se trouve à l'oblique.

21. Procédé selon l'une des revendications 17 à 19, **caractérisé en ce que**
l'angle d'inclinaison (ψ) est réglable.

22. Procédé selon l'une des revendications 17 à 21, **caractérisé en ce que**
le fond de la roue utilisé présente en plus du trou d'entraînement (112), un, plusieurs, ou de nombreux trous d'entraînement supplémentaires (112') dont les dimensions concordent avec celles du trou d'entraînement (112) et qui se trouvent tous avec le trou d'entraînement (112) sur un même cercle (KR).

23. Procédé selon l'une des revendications 17 à 22, **caractérisé en ce que**
l'on utilise un dispositif collecteur (152), notamment un tuyau flexible (152), un tube ou un conduit, lequel recueille les objets tombés vers le bas hors du trou d'entraînement (112) ou de l'un des trous supplémentaires (112'), à des fins d'acheminement ultérieur.

24. Procédé selon l'une des revendications 17 à 23, **caractérisé en ce que**
le fond de la roue (111) est conformé sur son côté tourné vers l'espace intérieur, de façon conoïde, conique ou convexe.

25. Procédé selon l'une des revendications 17 à 24, **caractérisé en ce que**
la roue convoyeuse (110) se met à vibrer tout du moins temporairement par l'action d'un vibrateur.

26. Procédé selon l'une des revendications 17 à 25, **caractérisé en ce que**
les dimensions du trou d'entraînement (112), respectivement du trou d'entraînement (112) et des trous d'entraînement supplémentaires (112') sont choisies de sorte que, au lieu que ce soit à chaque fois exactement l'un des objets, ce sont à chaque fois un nombre prédéfini d'exactement « n » objets qui tombent dans le trou d'entraînement (112), respectivement dans le trou d'entraînement (112) et dans les trous d'entraînement supplémentaires (112'), et peuvent ensuite tomber hors du trou d'entraînement (112), respectivement hors du trou d'entraînement supplémentaire (112'), de sorte que, au lieu d'être en mesure de séparer automatiquement les objets, le dispositif convoyeur est en mesure de regrouper automatiquement les objets en respectivement exactement « n » objets.

27. Procédé selon l'une des revendications 17 à 26, **caractérisé en ce que**
la vitesse de rotation de la roue convoyeuse (110) change.

28. Procédé selon l'une des revendications 17 à 27, **caractérisé en ce que**
la rotation de la roue convoyeuse (110) est arrêtée par intervalles et remise en marche.

29. Procédé selon l'une des revendications 17 à 28, **caractérisé en ce que**
le nombre des objets (8) éjectés est compté par un capteur de chocs.

30. Procédé selon l'une des revendications 17 à 29, **caractérisé en ce que**
l'on utilise un recouvrement supérieur (135), lequel
- est disposé dans l'espace intérieur parallèlement au fond de la roue (111) et n'est pas en mesure de participer à la rotation de la roue convoyeuse (110),
- est ou bien apposé au fond de la roue (111), ou bien disposé à une distance telle de celui-ci qu'elle est plus petite que le diamètre minima des objets (6,7,8,9),
- sert aussi de râcle pour les objets (9) se trouvant dans l'espace intérieur, et qui ne sont pas tombés dans le trou d'entraînement (112) ou l'un des trous supplémentaires (112').

31. Procédé selon l'une des revendications 17 à 30, **caractérisé en ce que**
l'on utilise comme moteur de rotation (114) un moteur qui est en mesure de conférer à la roue convoyeuse un mouvement de bascule par la commutation du sens de rotation réitéré successivement plusieurs fois.

32. Procédé selon l'une des revendications 17 à 31, **caractérisé en ce que**
l'on utilise comme fond de roue (111) un fond de roue interchangeable contre un fond de roue avec
- un trou d'entraînement ayant d'autres dimensions, ou
- des trous d'entraînement, respectivement des trous d'entraînement supplémentaires ayant d'autres dimensions, ou
- des trous d'entraînement, respectivement des trous d'entraînement supplémentaires en nombre différent.
